# FASCICULE DE BREVET EUROPEEN

(11) **EP 0 614 677 B1**
(45) Date de publication et mention de la délivrance du brevet: **16.01.2002**
(21) Numéro de dépôt: 94830102.3
(22) Date de dépôt: 07.03.1994
(51) Int. Cl.: A61N 1/368, A61N 1/05

(54) **Electrocathéter unique inséré à travers le sinus coronarien pour la stimulation cardiaque séquentielle (DDD)**
Elektrokatheter zur sequentiellen Herzreizung (DDD) mit einer durch den Sinus coronarius eingeführten einzigen Leitung
Electrocatheter for sequential heart pacing (DDD) with a single head positioned through the coronary sinus

(30) Priorité: 08.03.1993 IT FI930041
(43) Date de publication de la demande: 14.09.1994
(73) Titulaire: Cammilli, Leonardo, I-50100 Firenze (IT); Grassi, Gino, 50019 Sesto Fiorentino (Firenze) (IT)
(72) Inventeur: Cammilli, Leonardo, I-50100 Firenze (IT); Grassi, Gino, 50019 Sesto Fiorentino (Firenze) (IT)
(74) Mandataire: Martini, Lazzaro

(56) Documents cités:
- EP-A- 0 373 953
- WO-A-92/14512
- WO-A-92/18198
- US-A- 4 332 259
- US-A- 4 355 646

## Description

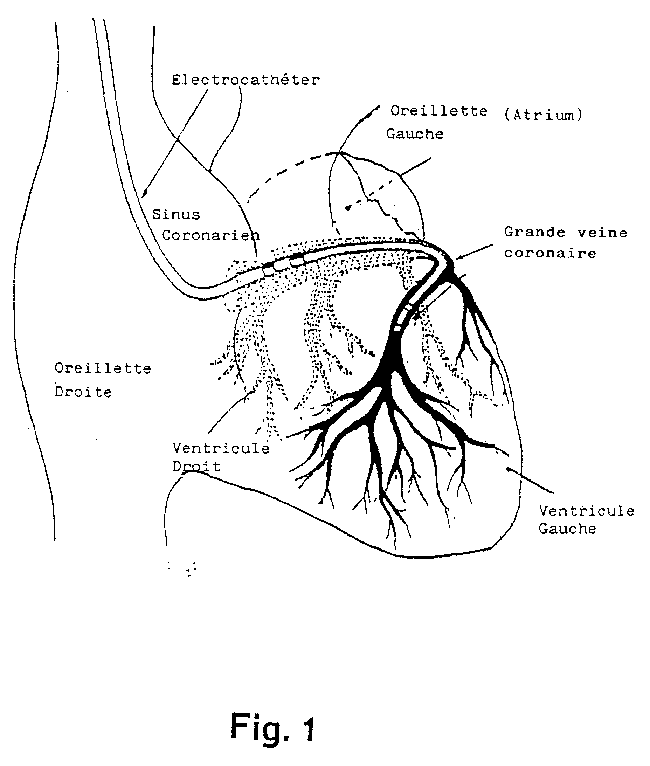

La présente invention a pour objet un système de stimulation cardiaque DDD ou DDDR (séquentiel en double chambre) qui utilise un seul cathéter qui supporte à la fois les électrodes d'oreillette (atrium) que celle ventriculaires et qui est positionné à travers le sinus coronarien comme schématisé sur la Fig. 1.
Le document WO-A-92/18198 décrit un défibrillateur cardiaque implantable comprenant une ou plusieures électrodes pour la défibrillation de l'oreillette (atrium) et une ou plusieurs électrodes ventriculaires pour relever l'activité electrique des ventricules. Ce document décrit un electrocathéter comme defini dans le préambule de la revendication 1.

La configuration des électrodes et la préformation du cathéter, qui seront décrites par la suite, sont telles que l'introduction de l'électrocathéter dans le sinus coronarien amène l'électrode (ou les électrodes) ventriculaire à s'insérer dans la grande veine coronaire de manière à établir un contact avec le ventricule gauche. Les électrodes d'oreillette (atrium) sont prédisposées à une distance qui leur permet de rester dans la partie moyenne du sinus coronarien, avec un bon contact vers l'oreillette (atrium) gauche.

L'expérimentation sur des animaux a démontré que le système permet des seuils de stimulation et de sensibilité aux signaux acceptables, superposables avec ceux trouvés dans les cathéters séparés dans l'auricole et dans le ventricule droits.
- la Fig. 1 montre schématiquement une possible application de la présente invention;
- la Fig. 2 représente un possible example de réalisation d'un électrocathéter selon la presente invention;
- la Fig. 3 représente un particulier de l'example de réalisation de Fig. 2;
- les Figg. 4 et 5 représentent deux possibles conformations d'une partie de l'example des figures précédentes.

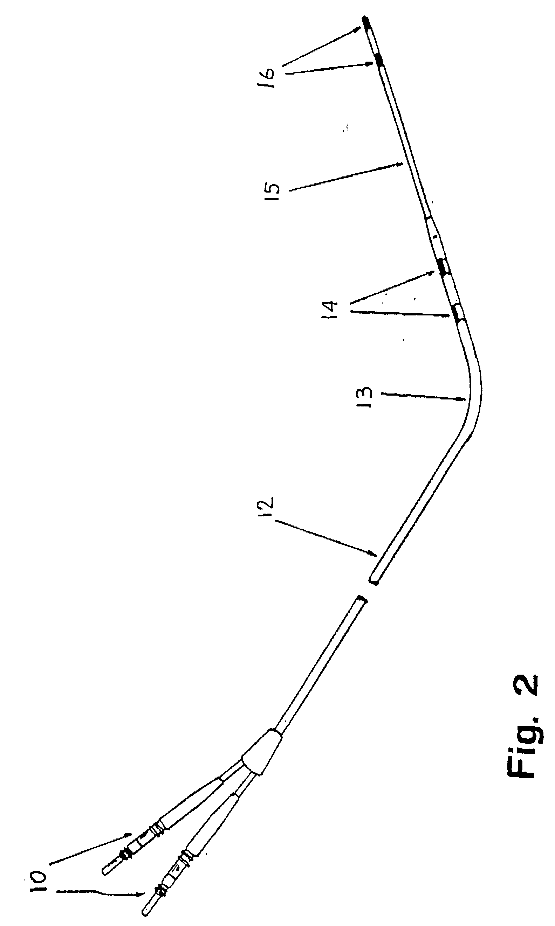

Le système proposé prévoit l'utilisation de pacemaker DDD ou DDDR normaux sans aucune adaptation, alors que le point crucial consiste dans l'électrocathéter et dans son positionnement. L'électrocathéter, dont la conformation générale est représentée sur la Fig. 2, est consitué par un unique cathéter (12 + 15) sur lequel sont positionnées les électrodes d'oreillette (atrium) 14 et celles ventriculaires 16.

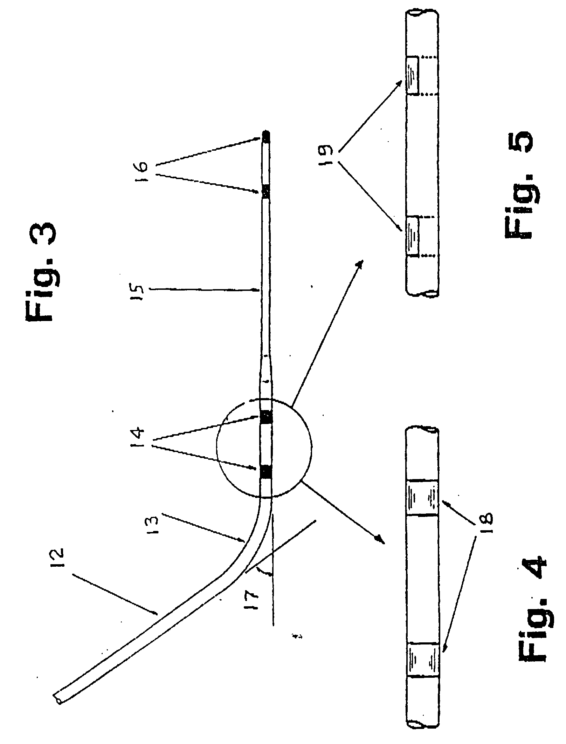

Ses caractéristiques principales sont illustrées dans les paragraphes suivants.
a) Le segment 15 qui supporte les électrodes (ou l'électrode) ventriculaire 16 doit avoir un diamètre non supérieur à 1,7 mm (5 Fr.) ou 2 mm (6 Fr.) et doit être très flexible, de manière à pouvoir être introduit dans la grande veine coronaire en suivant sa courbe autour du sillon oreillette-ventricule et ne pas l'occlure avec son volume.
   Dans ce but, on considère avantageux, mais pas limitatif, l'utilisation d'une seule électrode ventriculaire (en obtenant une stimulation unipolaire), de manière à pouvoir maintenir le calibre de la partie distale 15 du cathéter aux environs de 1,35 mm (4 Fr.). L'électrode (ou les électrodes) ventriculaire devra cependant rester à l'implantation dans la première moitié de la grande veine coronaire au-delà de la courbe qu'elle présente en direction de la pointe du ventricule gauche, où son diamètre intérieur est encore d'environ 3 - 4 mm.
   L'électrocathéter, de manière analogue à ceux utilisés pour le positionnement normal, sera réalisé de manière à pouvoir introduire à l'intérieur de celui-ci un mandrin qui le raidisse durant l'introduction dans la veine au moment de l'implantation. Ceci pourra être obtenu avec la technique connue consistant à utiliser comme conducteur une spirale multifilaire dans la lumière de laquelle le mandrin puisse passer.
b) A une distance à prédisposer entre 50 et 80 mm de l'électrode distale, le cathéter pourra changer de dimensions, de manière à pouvoir contenir également les conducteurs des électrodes d'oreillette 14. La partie 12 du cathéter pourra même arriver à un diamètre de 2,7 - 3 mm (8 - 9Fr.). Cette partie restera en effet dans le sinus coronarien, dont le diamètre intérieur est suffisamment grand (entre 8 et 15 mm).
   A la distance de 50 - 80 mm de la pointe du cathéter sera positionnée la première (distale) des deux électrodes d'oreillette (atrium) 14, et à une distance comprise entre 5 et 20 mm la seconde (proximale).
   A cause des différentes dimensions que peut avoir le coeur en fonction de l'âge, du sexe et d'autres facteurs, les électrocathéters réalisés pour le but de ce brevet devront être produits en différentes tailles à utiliser dans les différentes conditions. Les électrodes d'oreillette (atrium) (voir Fig. 4 et 5) pourront être conformées comme l'électrode 19, c'est-à-dire découvertes seulement radialement du côté orienté vers l'oreillette (atrium) et isolées du côté opposé; ou bien comme l'électrode 18, c'est-à-dire deux anneaux complètement conducteurs.
   La solution préférable, mais non limitative, est l'électrode 19, de manière à limiter la propagation du champ électrique dû à la stimulation en direction du ventricule et, en même temps, faciliter l'enregistrement des signaux provenant de l'oreillette (atrium) par rapport aux signaux ventriculaires.
   Bien que la configuration bipolaire avec deux électrodes (14) ait été indiquée comme préférable, il est également possible d'utiliser la configuration unipolaire dans laquelle seule une des deux électrodes indiquées sera utilisée et/ou présente sur le cathéter.
c) La partie 12 de l'électrocathéter présente une courbe préformée 13 à une distance de 15 à 25 mm de l'électrode d'oreillette (atrium) proximale de manière que l'angle 17 de la Fig. 3 soit dans un intervalle de 50° à 90°, avec une préférence non limitative à environ 60°.

Cette préformation a deux objectifs: le premier est de faciliter l'introduction dans le sinus coronarien; le second est de faire rester les électrodes d'oreillettes (atrium) de type 19 toujours orientées vers l'intérieur de la courbe et donc vers l'oreillette (atrium). Ces électrodes seront en effet fixées sur le cathéter dans cette position, de sorte que la partie conductrice découverte restera dirigée vers l'oreillette (atrium).
L'électrocathéter aura une longueur utile comparable à celle des endocavitaires normaux pour stimulation permanente: par exemple, sans limitation, entre 58 et 62 cm.
Dans la partie proximale du cathéter seront prédisposées les fiches de connexion compatibles avec le pacemaker sélectionné. Dans l'exemple non limitatif de la Fig. 2, on a pris comme hypothèse la solution la plus courante, c'est-à-dire deux fiches du type IS-1 bipolaires ou unipolaires, en fonction de la solution adoptée pour les électrodes d'oreillette (atrium) et ventriculaires. Il est cependant possible d'utiliser toutes les combinaisons et configurations requises par le pacemaker utilisé.

La conformation des conducteurs dans le cathéter pourra être réalisée, à titre d'exemple non limitatif, selon l'une des solutions suivantes. L'une d'elles utilise des tubes à plusieurs lumières en matériaux flexibles et biocompatibles (silicone, polyuréthane ou similaires): dans chaque lumière sera inséré un conducteur, de préférence constitué de fils enroulés en spirale, pour obtenir la résistance mécanique maximale. La spirale qui arrivera à l'électrode ventriculaire distale devra être telle qu'il soit possible d'insérer un mandrin de raidissement et de guidage du cathéter durant l'introduction dans la veine et le positionnement dans le coeur, comme cela est connu dans la pratique clinique d'implantation de stimulateurs cardiaques.

Une autre solution pourra consister dans l'utilisation de spirales coaxiales, isolées entre elles et à l'extérieur par des tubes avec parois minces, réalisés en matériaux flexibles, isolants et biocompatibles (polyuréthane, téflon, silicone ou similaires).
Le matérial des conducteurs devra présenter une résistance mécanique élevée et être biocompatible, comme MP35N, ELGILOY ou similaires.

L'introduction du cathéter par voie veineuse pourra s'effectuer en veine Céphalique, Jugulaire ou Sous-clavière, comme cela est déjà le cas pour les cathéters endocardes normaux.
Comme cela découle des recherches expérimentales, le système proposé présente des caractéristiques électrophysiologiques excellentes. Les seuils de stimulation se maintiennent à des valeurs comparables ou seulement légèrement supérieures à celles relevées dans le positionnement d'électrocathéters séparés dans l'oreillette (atrium) et le ventricule droits. Les signaux électriques cardiaques relevés sont eux aussi comparables à ceux habituels.
Les seuils de stimulation relevés varient de 0,5 à 1,5 Volt pour les ventriculaires et de 0,8 à 1,6 Volt pour les oreillettes (atrium).
Les signaux relevés sont compris entre 5 et 10 mV pour le ventricule et entre 1,2 et 2,8 mV pour l'oreillette.

## Revendications

1. Electrocathéter pour la stimulation cardiaque séquentielle, comprenant une ou plusieurs électrodes d'oreillette et une ou plusieurs électrodes ventriculaires destinées à être connectées à une pacemaker double chambre au moyen de connecteurs d'interface correspondants, opportunément conformé pour permettre son insertion dans le sinus coronarien de manière à positionner les électrodes d'oreillette dans le sinus coronarien en correspondance de l'oreillette gauche du coeur et les électrodes ventriculaires dans la grande veine coronaire au début de l'interventriculaire antérieure en correspondance du ventricule gauche; lesdites électrodes ventriculaires (16) étant disposées à l'extrémité distale du cathéter pour permettre la stimulation et le sondage ventriculaire et lesdites électrodes d'oreillette (14) étant opportunément espacées de celles ventriculaires (16) et situées en amont de celles-ci, pour permettre la stimulation et le sondage de l'oreillette gauche du coeur **caractérisé en ce qu'**il est préformé de manière à comprendre: - une première partie avec deux portions rectilignes dont les axes forment un angle (17) compris entre 50° et 90° et raccordées par une portion curviligne (13), la première portion rectiligne étant en correspondance des éléments (10) de connexion au pacemaker et la deuxième portion étant munie des électrodes d'oreillette (14); - une deuxième partie, de diamètre inférieur à la première, c'est-à-dire inférieur à 2mm, à l'extrémité libre de laquelle sont disposées les électrodes ventriculaires (16) et développée sur le prolongement de l'axe longitudinal de ladite portion contenant les électrodes d'oreillette (14).

2. Electrocathéter selon la revendication 1, **caractérisé en ce que** lesdites électrodes d'oreillette (14) sont disposés à une distance comprise entre 50 et 80 mm de l'extrémité libre du cathéter.

3. Electrocathéter selon la revendication 1, **caractérisé en ce que** lesdites électrodes d'oreillette (14) sont au nombre de deux: la distance entre les deux électrodes d'oreillette (14) étant comprise entre 5 et 20 mm.

4. Electrocathéter selon la revendication 1, **caractérisé en ce que** lesdites électrodes ventriculaires (16) sont au nombre de deux: la distance entre lesdites deux électrodes ventriculaires (16) étant comprise entre 5 et 20 mm.

5. Electrocathéter selon la revendication 1, **caractérisé en ce que** la surface active desdites électrodes d'oreillette (14) est orientée du même côté que la concavité (13) du cathéter.

6. Electrocathéter selon la revendication 1, **caractérisé en ce que** la surface active desdites électrodes d'oreillette (14) est développée en anneau, sur toute la circonférence du tronçon de cathéter correspondant.

7. Electrocathéter selon la revendication 1, **caractérisé en ce qu'**il comprend plusieurs conducteurs en forme de spirale multifilaire destinés à alimenter lesdites électrodes (14, 16) d'oreillette et ventriculaires, et **en ce qu'**il est pourvu d'un mandrin de guidage inséré dans la lumière de ladite spirale multifilaire pour permettre son raidissement et son guidage durant l'implantation.

8. Electrocathéter selon les revendications 1 et 7, **caractérisé en ce qu'**il est réalisé sous forme tubulaire, en matérial flexible pour permettre son insertion dans le sinus coronarien, et biocompatible, tel que le silicone ou polyuréthane, à l'intérieur duquel peut être inséré ladite spirale multifilaire.

9. Electrocathéter selon la revendication 1, **caractérisé en ce qu'**il est constitué par une pluralité de spirales métalliques coaxiales, lesquelles sont isolées entre elles et du milieu extérieur au moyen d'éléments tubulaires correspondants, en matérial flexible, isolant et biocompatible.

## Claims

1. Electrocatheter for sequential cardiostimulation, comprising one or more atrial electrodes and one or more ventricular electrodes connectable to a dual-chamber pace-maker by means of corresponding interface connectors, suitably shaped to allow the insertion thereof into the coronary sinus so as to position the atrial electrodes within the coronary sinus in correspondence of the left atrium of the heart and the ventricular electrodes within the great cardiac vein where the anterior interventricular begins in correspondence of the left ventricle, said ventricular electrodes (16) being located on the distal end of the catheter to allow for ventricular stimulation and sensing and the atrial electrodes (14) being suitably spaced from the ventricular ones (16) and located upstream of the latter, so as to allow for the heart left atrial stimulation and sensing **characterized in that** it comprises: - a first section with two straight portions which are angularly spaced by an angle (17) in a range of 50° to 90° and joined by a curvilinear portion (13), the first straight portion being in correspondence of the connectors (10) for the connection with the pace-maker and the second portion being provided with atrial electrodes (14); - a second section, of diameter lower than the first one, that is, less than 2 mm, at the free end of which the ventricular electrodes (16) are disposed and which develops on the extension of the longitudinal axis of said portion including the atrial electrodes (14).

2. Electrocatheter according to claim 1 **characterized in that** said atrial electrodes (14) are positioned at a distance of 50 to 80 mm from the tip of the catheter.

3. Electrocatheter according to claim 1 **characterized in that** the number of said atrial electrodes (14) is two: the distance between the two atrial electrodes (14) being comprised between 5 and 20 mm.

4. Electrocatheter according to claim 1 **characterized in that** the number of said ventricular electrodes (16) is two: the distance between the two ventricular electrodes (16) being comprised between 5 and 20 mm.

5. Electrocatheter according to claim 1 **characterized in that** the active surface of said atrial electrodes (14) is made to face the concavity (13) of the catheter.

6. Electrocatheter according to claim 1 **characterized in that** the active surface of said atrial electrodes (14) is ring-like developed, along the whole circumference of the corresponding length of the catheter.

7. Electrocatheter according to claim 1 **characterized in that** it comprises a plurality of conductors in the form of a multiwire spiral intended to power said atrial and ventricular electrodes (14, 16), and **in that** it is provided with a guide mandrel inserted said multiwire spiral to allow its stiffening and guiding during implantation.

8. Electrocatheter according to claims 1 and 7 **characterized in that** it has a tubular form, made of flexible material to allow its implantation within the coronary sinus, and biocompatible, such as silicone or polyurethane, within which may be inserted the said multiwire spiral.

9. Electrocatheter according to claim 1 **characterized in that** it is made by a plurality of metallic coaxial spirals, isolated to each other and to the outside by means of corresponding tubular elements, made in flexible, insulating and biocompatible material.

## Patentansprüche

1. Elektrokatheter für eine sequentielle Herzstimulation, der eine oder mehrere Herzvorkammer-Elektroden und eine oder mehrere Ventrikulär-Elektroden umfaßt, die dazu bestimmt sind, mit einem Doppelkammer-Herzschrittmacher mit Hilfe von entsprechenden Verbindungsleitungen verbunden zu werden, der in geeigneter Weise ausgebildet ist, um seine Implantation in den Koronarraum derart zu ermöglichen, daß die Vorkammer-Elektroden im Koronarraum im Bereich der linken Herzvorkammer und die Ventrikulär-Elektroden in der großen Konorarvene am Beginn der vorderen Herzscheidewand und im Bereich der linken Herzkammer positioniert werden, wobei die Ventrikulär-Elektroden (16) am distalen Ende des Katheters angeordnet sind, um eine ventrikuläre Stimulation und Sondierung zu ermöglichen, und wobei die Vorkammer-Elektroden (14) in geeigneter Weise von den Ventrikulär-Elektroden (16) im Abstand und stromauf von diesen angeordnet sind, um eine Stimulation und Sondierung der linken Herzvorkammer zu ermöglichen, **dadurch gekennzeichnet, daß** der Elektrokatheter folgende Bestandteile umfaßt:
- einen ersten Teil mit zwei geradlinigen Abschnitten, deren Achsen einen Winkel (17) zwischen 50° und 90° bilden und die durch einen gekrümmten Teil (13) miteinander verbunden sind, wobei sich der erste geradlinige Teil im Bereich der Verbindungselemente (10) mit dem Herzschrittmacher befindet und der zweite Teil mit den Vorkammer-Elektroden (14) bestückt ist,
- einen zweiten Teil mit einem kleineren Durchmesser als der erste Teil, d.h. weniger als 2 mm, an dessen freiem Ende die Ventrikulär-Elektroden (16) angeordnet sind und sich in Verlängerung der Längsachse des Teils erstrecken, der die Vorkammer-Elektroden (14) enthält.

2. Elektrokatheter nach Anspruch 1, **dadurch gekennzeichnet, daß** die Herzvorkammer-Elektroden (14) in einem Abstand zwischen 50 mm und 80 mm vom freien Ende des Katheters angeordnet sind.

3. Elektrokatheter nach Anspruch 1, **dadurch gekennzeichnet, daß** zwei Vorkammer-Elektroden (14) vorhanden sind, wobei der Abstand zwischen diesen beiden Vorkammer-Elektroden (14) zwischen 5 mm und 20 mm liegt.

4. Elektrokatheter nach Anspruch 1, **dadurch gekennzeichnet, daß** zwei Ventrikulär-Elektroden (16) vorhanden sind, wobei der Abstand zwischen diesen beiden Ventrikulär-Elektroden (16) zwischen 5 mm und 20 mm liegt.

5. Elektrokatheter nach Anspruch 1, **dadurch gekennzeichnet, daß** die aktive Oberfläche der Vorkammer-Elektroden (14) zur gleichen Seite wie die Konkavität (13) des Katheters hin orientiert ist.

6. Elektrokatheter nach Anspruch 1, **dadurch gekennzeichnet, daß** die aktive Oberfläche der Vorkammer-Elektroden (14) ringförmig über den gesamten Umfang des betreffenden Katheter-Abschnitts ausgebildet ist.

7. Elektrokatheter nach Anspruch 1, **dadurch gekennzeichnet, daß** er mehrere Leiter in Form einer multifilaren Spirale umfaßt, die dazu dienen, die Vorkammer- und Ventrikulär-Elektroden (14, 16) zu versorgen, und daß eine Führung vorgesehen ist, die in den inneren Freiraum der multifilaren Spirale eingeführt ist, um ihre Versteifung und Führung während der Implantation zu ermöglichen.

8. Elektrokatheter nach den Ansprüchen 1 und 7, **dadurch gekennzeichnet, daß** er in Form eines Rohres aus einem flexiblen Material gebildet ist, um seine Einführung in den Konorarraum zu ermöglichen, wobei es sich bei dem Material um ein biokompatibles Material wie z.B. Silikon oder Polyurethan handelt, in dessen Inneren die multifilare Spirale eingeführt werden kann.

9. Elektrokatheter nach Anspruch 1, **dadurch gekennzeichnet, daß** er von einer Vielzahl von koaxialen Metallspiralen gebildet wird, die gegeneinander und die äußere Umgebung mit Hilfe entsprechender schlauchförmiger Elemente aus einem flexiblen, isolierenden und biokompatiblen Material isoliert sind.
